# FASCICULE DE BREVET EUROPEEN

(11) **EP 1 689 356 B1**
(45) Date de publication et mention de la délivrance du brevet: **31.12.2008**
(21) Numéro de dépôt: 05717499.7
(22) Date de dépôt: 27.01.2005
(51) Int. Cl.: A61K 8/18, A61K 31/74

(54) **COMPOSITIONS TOPIQUES ASSOCIANT DES FRAGMENTS DE HYALURONATE DE SODIUM ET UN RETINOIDE**
TOPISCHE ZUSAMMENSETZUN MIT NATRIUM HYALURONAT FRAGMENTE UND EINEN RETINOID
TOPICAL COMPOSITIONS ASSOCIATING SODIUM HYALURONATE FRAGMENTS AND RETINOID

(30) Priorité: 29.01.2004 FR 0400826
(43) Date de publication de la demande: 16.08.2006
(60) Demande divisionnaire: 08167769.2
(73) Titulaire: Pierre Fabre Dermo-Cosmétique, 92100 Boulogne-Billancourt (FR)
(72) Inventeur: SAURAT, Jean-Hilaire, CH-1206 Genève (CH); KAYA, Gürkan, CH-1206 Genève (CH); BORDAT, Pascal, F-31320 Mervilla (FR)
(74) Mandataire: Warcoin, Jacques
(86) Numéro de dépôt international: PCT/FR2005/000176
(87) Numéro de publication internationale: WO 2005/082327

(56) Documents cités:
- EP-A- 0 138 572
- EP-A- 0 197 718
- WO-A-00/38647
- WO-A-01/03657
- WO-A-91/04279
- WO-A-93/16732
- WO-A-20/05039532
- GB-A- 2 228 736
- US-A- 5 977 088
- US-B1- 6 426 081
- DATABASE WPI Section Ch, Week 200267 Derwent Publications Ltd., London, GB; Class B05, AN 2001-430654 XP002330935 & KR 332 031 B (PACIFIC IND CO) 10 avril 2002 (2002-04-10)

## Description

La présente invention a pour objet des compositions topiques à base de hyaluronate, leur utilisation en cosmétologie et en dermatologie.

Le hyaluronate (HA) est la composante majeure de la matrice extracellulaire. Il est surtout présent dans les tissus conjonctifs dits « mous » par opposition à d'autres glycosaminoglycanes comme l'acide chondroïtine sulfurique présents dans les tissus dits « durs » tels que le cartilage. On le retrouve ainsi en quantités importantes principalement dans la peau.

Le HA est un glycosaminoglycane linéaire non sulfaté composé d'unités répétitives de D-acide glucuronique et de N-acetyl-D-glucosamine (Tammi R., Agren UM., Tuhkanen AL., Tammi M. Hyaluronan metabolism in skin. Progress in Histochemistry & Cytochemistry. 29(2):1-81, 1994).

Dans la peau normale, le HA est synthétisé essentiellement par les fibroblastes dermiques et les kératinocytes épidermiques (Tammi R., déjà cité). Grâce à ses résidus portant une charge négative, le HA joue le rôle d'une pompe à eau permettant de maintenir l'élasticité de la peau. Le HA a un rôle principal dans le contrôle de la diffusion des aliments, des hormones, des vitamines et des sels inorganiques du tissu conjonctif et dans le nettoyage des déchets métaboliques pouvant induire des réactions inflammatoires. Avec l'âge, la quantité de HA et son degré de polymérisation diminuent, résultant en une diminution de la quantité d'eau retenue dans le tissu conjonctif. La peau subit alors un processus de vieillissement qui aboutit à une augmentation de la fibrose et à une baisse de la teneur en fibres élastiques.

Dans la peau humaine normale, le HA existe sous forme d'un polymère de haut poids moléculaire (600 000 - 1 000 000 Da). La dégradation physiologique du HA dans la peau se fait par (i) l'internalisation par les kératinocytes via le CD44 et (ii) la fragmentation intracellulaire en fragments de taille intermédiaire par les hyaluronidases (60 000-300 000 Da). Le HA fragmenté est relâché par les kératinocytes, passe la membrane basale et est libéré directement dans les vaisseaux lymphatiques (Tammi R. et al., déjà cité).

Dans des conditions inflammatoires, l'accumulation des formes de bas poids moléculaire de HA a été démontrée chez l'animal. Pendant l'inflammation, des facteurs chimiotactiques plaquettaires comme de la fibrine stimulent l'afflux et l'activation des fibroblastes qui dégradent le HA par sécrétion de hyaluronidase résultant en des concentrations tissulaires élevées de petits fragments de HA. La génération de ces petits fragments de HA se fait aussi par divers mécanismes comme la dépolymérisation par les espèces réactives d'oxygène relâchées par les granulocytes, ou dans la peau irradiée par les ultraviolets, ou la synthèse de *novo* de fragments de bas poids moléculaire. Plusieurs études ont suggéré que le HA de haut et bas poids moléculaire peut avoir des effets biologiques.différents sur les cellules et les tissus (McKee CM., Penno MB., Cowman M., Burdick MD., Strieter RM., Bao C., Noble PW. Hyaluronan (HA) fragments induce chemokine gene expression in alveolar macrophages. The role of HA size and CD44. Journal of Clinical Investigation. 98(10):2403-13, 1996 ; Termeer CC., Hennies J., Voith U., Ahrens T., Weiss JM., Prehm P., Simon JC. Oligosaccharides of hyaluronan are potent activators of dendritic cells. Journal of Immunology. 165(4):1863-70, 2000 ; Fitzgerald KA., Bowie AG., Skeffington BS., O'Neill LA., Ras, Protein kinase C zeta, and I kappa B kinases 1 and 2 are downstream effectors of CD44 during the activation of NF-kappa B by hyaluronic acid fragments in T-24 carcinoma cells. Journal of Immunology. 164 (4) : 2053-63, 2000).

Il a été démontré que le HA de poids moléculaires intermédiaires (50 000 - 250 000 Da), appliqué sur la peau murine et humaine, traverse les couches épidermiques et dermiques. Les produits de dégradation apparaissent dans le sérum 2 heures après l'application du HA sur la peau. Le poids moléculaire du HA récupéré dans le sérum est légèrement inférieur à celui du HA appliqué sur la peau, démontrant ainsi que le passage transcutané du HA ne se limite pas uniquement à des fragments de plus petite taille (100 - 10 000 Da) (Brown TJ., Alcorn D, Fraser JR. Absorption of hyaluronan applied to the surface of intact skin. Journal of Investigative Dermatology. 113(5):740-6, 1999).

La vitamine A (rétinol) et ses dérivés naturels et synthétiques, collectivement désignés comme rétinoïdes, constituent une grande variété de substances ayant des effets marqués notamment dans l'embryogénèse, la reproduction, le mécanisme de la vision, la régulation de la croissance et le phénomène de différenciation cellulaire (Blomhoff R. et al. 1991, Physiol. Rev. 71:951-990 ; Sporn M.B. et al. (1994) in the Retinoids, 2nd Ed. Raven Press, NY).

Les rétinoïdes naturels sont définis par la formule générale (I) suivante telle que définie dans (IUPAC-IUB Joint Commission on Biochemical Nomenclature in Eur. J. Biochem. (1982), 129, 1-5) : 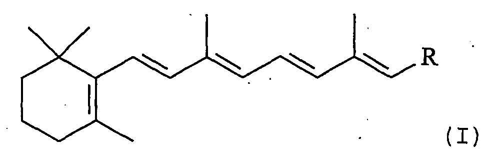 dans laquelle
R = CH₂OH correspond au rétinol (ROL)
R = CHO correspond au rétinal (RAL)
R = COOH correspond à l'acide rétinoïque (RA).

Les effets biologiques des rétinoïdes sont médiés par leur interaction avec les récepteurs nucléaires du type RAR (récepteur de l'acide rétinoïque) et RXR (récepteur X de l'acide rétinoïque). Le ligand connu des récepteurs RARs est l'isomère trans de la forme acide de la vitamine A (acide trans-rétinoïque).

Dans beaucoup de types cellulaires, y compris les cellules épithéliales, le ROL est métabolisé en rétinylesters.

L'importance des rétinoïdes en dermatologie remonte aux premières observations chez l'animal d'anomalies cutanées induites par un déficit en vitamine A (Wolbach et Howe, J. exp. Med. 43 : 753).

Aujourd'hui, de très nombreux rétinoïdes ont été synthétisés. Les domaines principaux d'utilisation restent les indications dermatologiques telles que les kératoses actiniques, l'acné et d'une façon générale le traitement par voie topique ou orale des désordres de kératinisation tels que le psoriasis et la maladie de Darier.

Par ailleurs, un certain nombre de combinaisons ou d'associations avec des rétinoïdes sont connues :
- combinaisons de molécules agissant sur la différenciation cellulaire et l'immunomodulation, par exemple combinaison de rétinoïdes et de chimiothérapie dans les lymphomes cutanés (Thestrup-Petersen et al. Br. J. Dermatol. 118 : 811-818),
- combinaison de rétinoïdes et de photothérapie par exemple en association avec les psoralènes (Saurat et al. Dermatologica 177 : 218-224)
- combinaison de molécules affectant le métabolisme des rétinoïdes. C'est par exemple le cas de l'interaction entre les azolés et les dérivés de la vitamine D inhibant le métabolisme des rétinoïdes et permettant d'augmenter les taux intracellulaires d'hormone active (Kato et al. Biochem. J. (1992) 286 : 755-760, Jugert et al. Skin Pharmacol. 1998).

Il est connu par ailleurs que l'un des effets secondaires majeurs des rétinoïdes lors d'une application topique reste l'irritation induite. Cet effet peut-être rédhibitoire à l'observance du traitement.

Le rétinaldehyde (RAL), un membre de la famille des rétinoïdes, est un métabolite naturel de la vitamine A, actuellement utilisé en application topique chez l'homme. Il a été démontré que le RAL exerce une activité biologique dans la peau en induisant une hyperplasie épidermique, de même qu'une nette augmentation de l'expression du CD44 et du HA dans l'épiderme folliculaire et interfolliculaire des souris C57BL/6 et SKH1 sans poils (SKH1 *hairless*)*.* Ces effets ont également été observés suite à l'application topique de l'acide rétinoïque (RA) et du rétinol (ROL). Toutefois, l'expression du CD44 et celle du HA ont été plus fortement augmentées chez les souris traitées au RAL que chez celles traitées au RA ou au ROL.

Le CD44 est une glycoprotéine transmembranaire polymorphique qui a plusieurs isoformes générées par l'épissage alternatif et les modifications post-traductionnelles. Dans une étude récente nous avons démontré que deux fonctions majeures du CD44 dans la peau murine sont (i) la régulation de la prolifération kératinocytaire en réponse à des stimuli extracellulaires et (ii) le maintien de l'homéostase locale du HA (Kaya G., Rodriguez I., Jorcano JL., Vassalli P., Stamenkovic I. Selective suppression of CD44 in keratinocytes of mice bearing an antisense CD44 transgene driven by a tissue-specific promoter disrupts hyaluronate metabolism in the skin and impairs keratinocyte proliferation. Genes & Development. 11(8):996-1007, 1997). Nous avons également observé une diminution de l'expression du CD44 épidermique chez les patients souffrant de lichen scléro-atrophique, qui est potentiellement responsable de la déposition dermique du HA et de l'atrophie épidermique dans cette maladie (Kaya G., Augsburger E., Stamenkovic K., Saurat JH. Decrease in epidermal CD44 expression as a potential mechanism for abnormal hyaluronate accumulation in superficial dermis in lichen sclerosus and atrophicus. Journal of Investigate Dermatology. 115 (6) : 1054-8, 2000).

Le CD44 est impliqué dans les interactions cellule - cellule et cellule - matrice. Une étude récente met en évidence que le couple formé par le CD44 et les fragments de HA (CD44-HAF) est inducteur de mitoses et de néosynthèse de HA (Laurent TC, Laurent UB, Fraser JR. The structure and function of hyaluronan: An overview. Immunol Cell Biol 79(2):1-7, 1996). Les effets épidermiques et dermiques du HA et du RAL semblent donc être médiés par le CD44.

Actuellement il existe des préparations pharmaceutiques et cosmétiques contenant des sels inorganiques du HA de haut poids moléculaire, notamment Healon, Hyalgan, Provisc, Vitrax, et ceux cités dans Martindale The Complete Drug reference, 32ème édition, 1999, The Pharmaceutical Press Editor.

Toutefois, le HA passe difficilement à travers la peau étant donné son poids moléculaire élevé.

C'est pourquoi, dans la demande internationale WO 02/076470 A1, les auteurs proposent une composition associant de la N-acétyl-glucosamine à un rétinoïde. Cette combinaison permet d'augmenter de façon synergique la synthèse d'acide hyaluronique par les cellules épidermiques *in vitro.* Aucun résultat obtenu après application de la formulation *in vivo* n'est mentionné.

La demande japonaise 11279042 décrit des compositions à base de fragments d'acide hyaluronique sulfaté, lesdits fragments ayant de préférence un poids moléculaire compris entre 1 000 et 50 000 Da, les groupement sulfates occupant 10 à 90 % de l'ensemble des substituant R1, R2, R3 et R4 dans la formule. Ces fragments de bas poids moléculaire sont très actifs pour maintenir l'élasticité de la peau et éviter la kératinisation. Au contraire, des fragments d'acide hyaluronique non sulfatés se sont révélés inactifs dans le test.

La demande internationale WO 01/03657 A1 décrit une composition permettant d'éviter le vieillissement prématuré de la peau en bonne condition, contenant du rétinol et un hyaluronate de poids moléculaire compris entre 1 et 1 000 000 Da.

La demande internationale WO 2005/039532 A1 décrit des compositions, pouvant s'appliquer de façon topique, sous forme de micro émulsions et contenant un rétinoïde tel que l'acide 13-cis-rétinoïque comme agent actif en combinaison avec un dérivé d'acide hyalutonique ayant une masse moléculaire comprise, de préférence, entre 400 et 1 000 000 Da et plus particulièrement entre 400 et 200 000 Da.

La demande EP 0 197 718 décrit l'utilisation de fragments hyaluroniques de poids moléculaire compris entre 50 000 et 750 000 Da en association avec une autre substance active dermatologiquement.

Or les inventeurs ont montré de manière surprenante que le HA non sulfaté hydrolysé en des fragments de poids moléculaires compris entre 50 000 et 750 000 Da possède une activité biologique sur la peau qui est amplifié lorsque ces fragments sont associés au rétinal.

La présente invention a en conséquence pour objet des compositions destinées à l'application topique caractérisées en ce qu'elles comprennent à titre de principe actif un ou plusieurs fragments de hyaluronate de bas poids moléculaire compris entre 50 000 et 750 000 Da et en ce qu'elles comprennent en outre du rétinal.

Dans un mode de réalisation préféré de l'invention, le poids moléculaire des fragments de hyaluronate est compris entre 50 000 et 250 000 Da ou entre 250 000 et 750 000 Da.

La combinaison de fragments de hyaluronate d'un poids moléculaire compris entre 50 000 et 750 000 Da avec le rétinal a un effet synergique sur la synthèse de l'acide hyaluronique par les kératinocytes.

On entend par hyaluronate n'importe quel sel, notamment le hyaluronate de sodium.

Les compositions topiques selon l'invention peuvent contenir également des colorants, des huiles silicones, des rétinoïdes ou des pigments colorants, des antiseptiques, des huiles végétales, des antioxydants, des sels minéraux, des épaississants, des modificateurs de pH, des agents absorbant les rayons ultraviolets, des vitamines ou tout autre excipient acceptable en dermatologie et en pharmacie.

Les compositions selon l'invention peuvent être utilisées en cosmétologie et en dermatologie pour prévenir ou améliorer les peaux ridées, les peaux sèches... et permettent de maintenir la fermeté et l'humidité de la peau.

Les compositions selon l'invention peuvent être utilisées dans des préparations topiques, dans le domaine de la dermatologie ou de la cosmétologie, dans le but de prévenir ou de traiter les dermatoses associées à une atrophie du tissu cutané et pour lesquelles il sera nécessaire d'améliorer l'état d'hydratation de la peau, de diminuer l'atrophie cutanée comme par exemple dans les effets secondaires aux traitements corticoïdes, de diminuer les rides, de lutter contre le vieillissement cutané photoinduit ou non, de relancer l'activité cellulaire épidermique et dermique, de raffermir la peau, d'augmenter son élasticité.

L'invention est illustrée par les exemples et figures qui suivent.

La figure 1 représente la quantité de HA mesurée dans l'épiderme chez des animaux non traités ou des animaux traités deux fois par jour par la préparation 5, la préparation 3 ou la préparation 7 obtenues selon les modes opératoires décrits dans les exemples 1 et 2.

La figure 2 représente le dosage du HA dans le derme chez des animaux non traités ou traités avec la préparation 5, 3 ou 7 telles que préparées dans les exemples 1 et 2.

### EXEMPLE COMPARATIF 1: Effet des fragments de hyaluronate utilisés par voie topique

### 1. MATERIELS ET METHODES

### 1.1. Préparations

3 types de fragments de hyaluronate (HAF) ont été évalués:
● HAF de très haut poids moléculaire (1 000 000-2 000 000 Da)
● HAF de bas poids moléculaire (50 000-750 000 Da)
   - HAF (250 000-750 000 Da)
   - HAF (50 000-250 000 Da)
● HAF de très bas poids moléculaire (1 000-20 000 Da)
Ces fragments ont été inclus dans les préparations cosmétiques classiques, dont deux exemples de composition sont donnés dans le tableau 1 ci-après :

| Ingrédients | Quantités % | Quantités % |
|---|---|---|
| PEG 600 | 5 | - |
| PEG 400 | - | 1 |
| Sorbitol | - | 2 |
| glycérine | - | 10 |
| BHT | 0,02 | - |
| Lauryl sulfate de sodium | - | 0,25 |
| Carbopol | 1 | - |
| Vitamine E acétate | 0,5 | - |
| Blanc de baleine | - | 6 |
| Alcool cétostéarylique | - | 3 |
| Huile de vaseline épaisse | 5 | - |
| Crémophor RH 40 | 2 | - |
| Acide sorbique | 0,05 | - |
| Nipagine | 0,15 | - |
| Phenonip | | 1 |
| Triéthanolamine | 2,48 | - |
| Eau | qsp 100 | qsp 100 |

### 1.2. Mesure de l'activité des HAF

On utilise des souris SKH1 sans poils (SKH1 *hairless*) à raison de 3 animaux par groupe.

Les différentes préparations sont appliquées par voie topique.

L'épaisseur épidermique est mesurée par un oculaire gradué (Zeiss); grossissement 40 fois, on fait la moyenne de 5 champs par souris.

La cellularité dermique est évaluée en calculant le nombre de cellules dermiques, grossissement 40 fois, on fait la moyenne de 5 champs par souris.

Le nombre des cellules prolifératives de l'épiderme et du derme est mis en évidence par l'immunomarquage de Ki 67 avec un anticorps anti Ki-67 (rat anti-souris, Dako).

La présence de hyaluronate dans le derme est mesurée après application des différentes préparations à raison de deux applications par jour selon la technique ELISA (Corgenix) et le hyaluronate est mis en évidence sur des coupes histologiques par la coloration au fer colloïdal.

### 2. RÉSULTATS

### 2.1. Effets sur l'épaisseur épidermique, la cellularité dermique et le nombre de cellules prolifératives

Les résultats sont rassemblés dans les tableaux 2 et 3.

Les préparations 2 et 3 formulées avec des HAF de bas poids moléculaire selon l'invention et appliquées par voie topique, augmentent de façon significative l'épaisseur épidermique et la cellularité dermique chez la souris SKH1 sans poils (SKH1 *hairless*)*,* par rapports aux préparations 1 et 4 contentant respectivement des HAF de haut et très bas poids moléculaire (tableau 2). La préparation 3 augmente aussi de façon significative le nombre des cellules prolifératives de l'épiderme et du dermes chez la souris SKH1 sans poils (SK1 *hairless*)*,* par rapport aux préparations 1 et 4 (tableaux 3).

L'application topique des préparations 2 and 3 induit une cellularité augmentée dans le derme superficiel et profond. Les cellules qui sont augmentées en nombre sont principalement des fibroblastes qui présentent un phénotype d'activation avec un réticulum endoplasmique bien développé.

### 2.2. Effets sur le taux de HA dans le derme

Les résultats sont rassemblés dans le tableau 4.

La présence de HA dans le derme a été détectée pour les préparations 2 et 3 (préparation 3 > préparation 2).

Le taux de HA n'est pas détectable après application des préparations 1 et 4.

Ainsi les inventeurs ont montré que l'application topique de HAF de bas poids moléculaire a deux conséquences majeures :
1-L'hyperplasie épidermique importante accompagnée par une augmentation de la prolifération cellulaire.
2-L'accumulation focale du HA dans le derme superficiel avec une augmentation importante du nombre de fibroblastes:
conséquences qui ne sont pas observés avec les HAF de haut ou de très bas poids moléculaires.

### EXEMPLE 2: Effet de l'association du rétinal avec des HAF de bas poids moléculaire

### 1. MATÉRIELS ET MÉTHODES

Les fragments du tableau 5 sont inclus dans les préparations cosmétiques classiques telles qu'exemplifiées précédemment.

### 2. RÉSULTATS

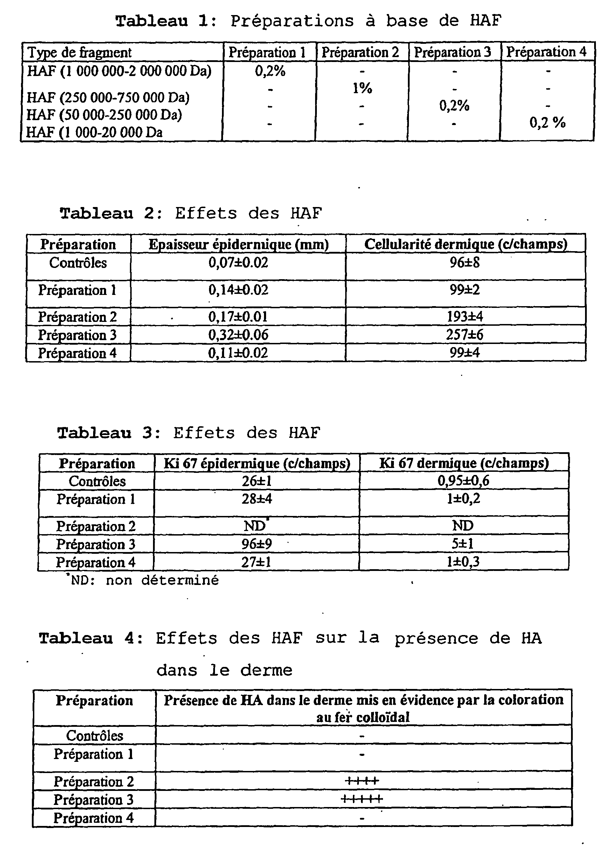

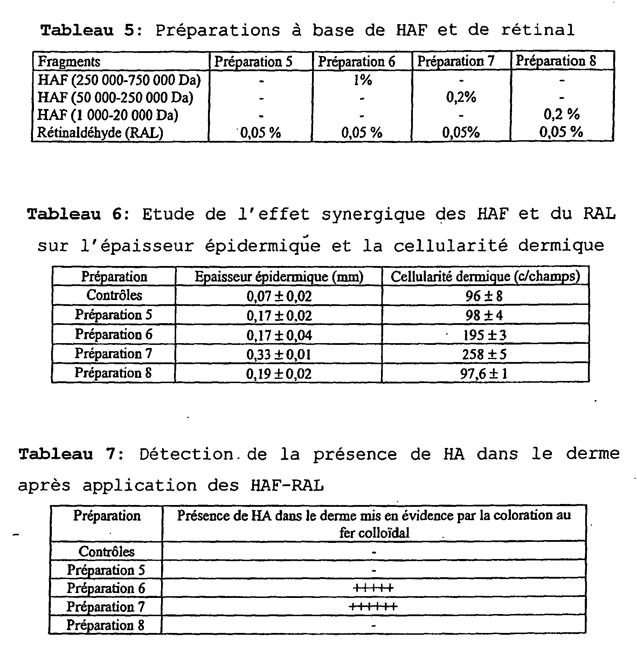

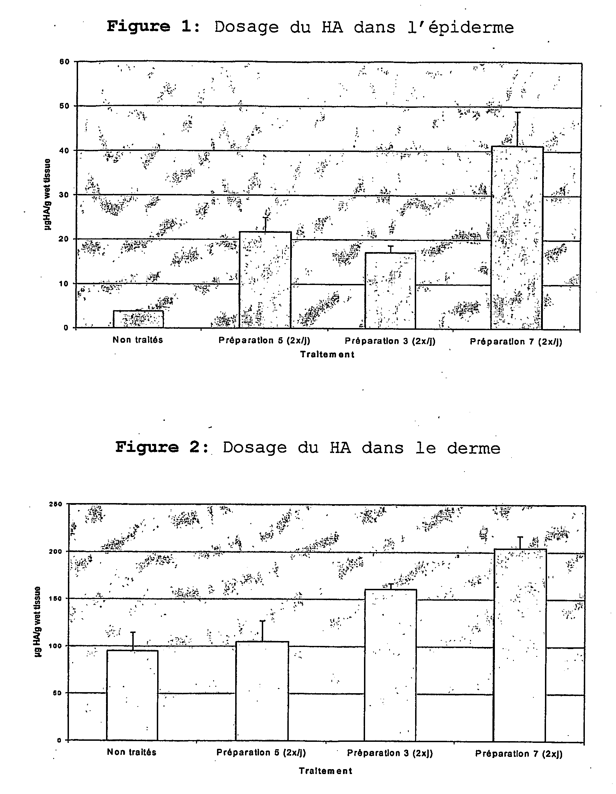

Ils sont rassemblés dans les tableaux 6 et 7 et dans les figures 1 et 2. L'application topique des préparations 6 et 7 augmente le nombre du foyers d'épiderme épaissi.

L'effet de l'application du RAL seul (préparation 5) est comparable à l'effet obtenu avec la préparation 8 associant du HAF de petite taille moléculaire avec le RAL, preuve qu'il n'existe pas de synergie entre RAL et des fragments de très bas poids moléculaire.

L'effet synergique de l'association HAF-RAL est particulièrement visible au niveau de la cellularité dermique pour les préparations 6 et 7 (cf tableau 5).

L'application topique des préparations 6 et 7 augmente également la coloration de HA dermique (préparation 7> préparation 6) (cf tableau 7).

L'effet synergique de l'association HAF-RAL a également été mis en évidence par dosage ELISA de la présence de HA dans le derme et l'épiderme des souris qui ont été traitées par les différentes préparations mentionnées ci-dessus.

Les résultats montrent une augmentation significative de la production de HA aussi bien dans le derme (figure 2) que dans l'épiderme (figure 1) après traitement par l'association HAF-RAL (préparation 7) comparativement au traitement par RAL seul (préparation 5) ou HAF seul (préparation 3).

## Revendications

1. Compositions destinées à l'application topique **caractérisées en ce qu'**elles comprennent à titre de principe actif un ou plusieurs fragment(s) de hyaluronate dont le poids moléculaire est compris entre 50 000 et 750 000 Da et **en ce qu'**elles contiennent en outre du rétinal.

2. Compositions selon la revendication 1 **caractérisées en ce que** le poids moléculaire des fragments de hyaluronate est compris entre 50 000 et 250 000 Da ou entre 250 000 et 750 000 Da.

3. Utilisation d'une composition selon l'une quelconque des revendications 1 à 2 en cosmétologie.

4. Composition selon l'une quelconque des revendications 1 à 3 à titre de médicament utilisable en dermatologie.

5. Composition pharmaceutique **caractérisée en ce qu'**elle contient à titre de principe actif un ou plusieurs fragment(s) de hyaluronate dont le poids moléculaire est compris entre 50 000 et 750 000 Da et **en ce qu'**elles contiennent en outre du rétinal associés à des excipients pharmaceutiquement acceptables.

6. Utilisation d'une composition selon l'une quelconque des revendications 1 et 2, ou 4 et 5, pour la préparation d'un médicament destiné au traitement ou à la prévention de l'atrophie du tissu cutané, du vieillissement cutané photo induit ou non.

7. Utilisation selon la revendication 3 **caractérisée en ce que** la composition est utilisée pour l'atrophie du tissu cutané, l'amélioration de l'état d'hydratation de la peau, la diminution des rides ou le raffermissement de la peau.

## Claims

1. Compositions intended for topical application, **characterized in that** they comprise as an active ingredient one or more fragments of hyaluronate, the molecular weight of which is comprised between 50,000 and 750,000 Da and **in that** they further contain retinal.

2. The compositions according to claim 1, **characterized in that** the molecular weight of the hyaluronate fragments is comprised between 50,000 and 250,000 Da or between 250,000 and 750,000 Da.

3. The use of a composition according to any of claims 1 to 2 in cosmetology.

4. The composition according any of claims 1 to 3, as a drug which may be used in dermatology.

5. A pharmaceutical composition, **characterized in that** it contains as an active ingredient, one or more fragments of hyaluronate, the molecular weight of which is comprised between 50,000 and 750,000 Da and **in that** they further contain retinal, associated with pharmaceutically acceptable excipients.

6. The use of a composition according to any of claims 1 and 2, or 4 and 5, for preparing a drug intended for treating or preventing atrophy of skin tissue, skin ageing whether photo-induced or not.

7. The use according to claim 3, **characterized in that** the composition is used for atrophy of skin tissue, improvement of the hydration condition of the skin, reduction of wrinkles or skin firming.

## Patentansprüche

1. Zusammensetzungen zur topischen Anwendung, **dadurch gekennzeichnet, dass** sie als Wirkstoff ein oder mehrere Hyaluronatfragmente umfassen, deren Molekulargewicht zwischen 50000 und 750000 Da inklusive ist und **dadurch**, dass sie weiterhin Retinal enthalten.

2. Zusammensetzungen nach Anspruch 1, **dadurch gekennzeichnet, dass** das Molekulargewicht der Hyaluronatfragmente zwischen 50000 und 250000 Da oder zwischen 250000 und 750000 Da inklusive ist.

3. Verwendung einer Zusammensetzung nach einem der Ansprüche 1 bis 2 in der Kosmetologie.

4. Zusammensetzung nach einem der Ansprüche 1 bis 3 als in der Dermatologie anwendbares Arzneimittel.

5. Pharmazeutische Zusammensetzung, **dadurch gekennzeichnet, dass** sie als Wirkstoff ein oder mehrere Hyaluronatfragmente enthält, deren Molekulargewicht zwischen 50000 und 750000 Da inklusive ist und **dadurch**, dass sie weiterhin Retinal enthält, verbunden mit pharmazeutisch akzeptablen Trägerstoffen.

6. Verwendung einer Zusammensetzung nach einem der Ansprüche 1 und 2 oder 4 und 5 zur Zubereitung eines Arzneimittels, das zur Behandlung oder Vorbeugung von Atrophie des Hautgewebes, der durch oder nicht durch Licht induzierten Hautalterung bestimmt ist.

7. Verwendung nach Anspruch 3, **dadurch gekennzeichnet, dass** die Zusammensetzung bei Atrophie des Hautgewebes, zur Verbesserung des Hydratisierungszustands der Haut, zur Verringerung von Falten oder zur Straffung der Haut verwendet wird.
